# EUROPEAN PATENT APPLICATION

(11) **EP 2 133 433 A1**
(43) Date of publication of application: **16.12.2009**
(21) Application number: 08305241.5
(22) Date of filing: 09.06.2008
(51) Int. Cl.: C12Q 1/68

(54) **A method for predicting responsiveness to a treatment with an anti-HER2 antibody**

(71) Applicant: Centre Georges François Leclerc, 21079 Dijon Cedex (FR)
(72) Inventor: The designation of the inventor has not yet been filed
(74) Representative: Chajmowicz, Marion

(57) **Abstract**

The invention provides an *in vitro* method for predicting whether a patient would be responsive to a treatment with an anti-HER2 blocking agent, such as trastuzumab, which method comprises determining the expression level of at least 4 genes in a biological sample of said patient, wherein said genes are GPR22, PEX19, GRHL2 and DERL1. The invention further provides a DNA chip for performing such method.

## Description

The present invention relates to a method for predicting the response to a treatment with a HER2 blocking agent, such as an anti-HER2 antibody.

The human epidermal growth factor receptor 2 (HER2) proto-oncogene encodes a 185 kDa glycoprotein receptor tyrosine kinase, which is a member of the growth factor receptor family that includes epidermal growth factor receptor (EGFR) 1, 3, and 4. Amplification and overexpression of HER2 is observed in 20-30% of invasive breast cancer and correlates with tumor progression and poor prognosis. Although the EGFR family stimulates mitogenesis through ligand-induced pathways, there is no known ligand for HER2. Increased HER2 expression induces a signaling pathway that involves Ras and Src as well as PI3K/Akt and is associated with tumor formation. The transforming ability of HER2 has been linked to cell survival and through mitogenic signaling pathways.

Trastuzumab (Herceptin®; F. Hoffmann-La Roche, Basel, Switzerland) is a humanized monoclonal antibody directed against the HER2 protein. It produces significant (>50%) tumor regression in ∼15% of patients with HER2-positive metastatic breast cancer that is refractory to conventional therapy, and in ∼23% of patients when used as first-line therapy (Cobleigh et al, 1999). The addition of trastuzumab to standard chemotherapy significantly improves response rate, response duration, and survival. The clinical benefits of trastuzumab-based therapies have been well documented in both the adjuvant and the metastatic settings.

However resistance to trastuzumab is often observed in women with HER2-positive breast cancer and has been shown to involve multiple potential mechanisms.

The precise molecular pathways through which trastuzumab exerts its antitumor effects in breast cancer cells, or through which a patient shows resistance to these antitumor effects, are not yet fully understood.

Trastuzumab action involves multiple mechanisms including the induction of apoptotic signaling pathways, cell cycle perturbation, cellular cytotoxicity, and inhibition of nuclear excision repair mechanisms. Treatment with trastuzumab dephosphorylates and down-regulates HER2, leading to significant clinical efficacy against HER2-positive breast cancer. It also sensitizes breast cancer cells to chemotherapeutic agents, especially tubulin-polymerizing agents and radiation therapy. It was demonstrated that anti-HER2 monoclonal antibodies inhibit HER2-overexpressing breast cancer cells through G1 cell cycle arrest that was associated with the induction of the cyclin-dependent kinase (CDK) inhibitor p27kip1 and reduction of CDK2. Trastuzumab may also inhibit the PI3K/Akt pathway by promoting PTEN activation. Overexpression of HER2 in human tumor cells has also been shown to be associated with increased angiogenesis and expression of vascular endothelial growth factor, and trastuzumab has been shown to reduce tumor volume and microvessel density in HER2-positive breast cancer models in vivo. Synergy with DNA-damaging drugs is thought to be due to trastuzumab-mediated inhibition of DNA repair. Trastuzumab partially inhibits repair of DNA adducts in vitro after treatment with cisplatin and blocks unscheduled DNA synthesis after radiation. Finally, trastuzumab has also been shown to be associated with immunoreactive actions via antibody-directed cellular cytotoxicity (ADCC).

Recently, trastuzumab-based neoadjuvant chemotherapy has been shown to achieve promising efficacy, with a good pathological complete response (pCR) rate, while being well tolerated in women with stage II or III HER2-positive breast cancer (Buzdar et al, 2005; Coudert et al, 2006; Coudert et al, 2007). Among taxanes, docetaxel associated with trastuzumab shows evidence of improved efficacy in obtaining pCR rates.

To date, only one study has used RNA profiling to predict responses to trastuzumab-vinorelbine-based treatments in patients with early HER2-positive breast cancer (Harris et al, 2007). In this study, resistant tumors exhibited a higher expression of several growth factors, growth factor receptors, the PI3K regulatory subunit p85, microtubule-associated protein 2, and some basal genes.

There is still a need for a complete molecular signature which would be useful for predicting responsiveness to an anti-HER2 antibody treatment, especially a trastuzumab-docetaxel-based chemotherapy.

### Summary of the invention

Using microarray analysis, the inventors performed a RNA profiling and found a signature of pathological complete response (pCR).

On this basis the invention provides an *in vitro* method for predicting whether a patient would be responsive to a treatment with a HER2 blocking agent, preferably an anti-HER2 antibody such as trastuzumab, which method comprises determining the expression level of at least 4 genes in a biological sample of said patient, wherein said genes are GPR22, PEX19, GRHL2 and DERL1.

In a particular embodiment, the invention provides an *in vitro* method for predicting whether a patient would be responsive to a treatment with a combination of trastuzumab and docetaxel, which method comprises determining the expression level of at least 4 genes in a biological sample of said patient, wherein said genes are GPR22, PEX19, GRHL2 and DERL1.

The combined expression profile of these genes is informative of the status of the patient who, before any treatment with a HER2 blocking agent, can be classified as responder or non-responder, and be given the appropriate treatment.

The method usually comprises the further step of comparing the expression level of said genes with reference values obtained from responder and non-responder groups of patients.

Generally speaking, the patient is preferably affected with a HER2-positive cancer. The patient is preferably with breast cancer.

The expression level is advantageously determined by quantifying the level of mRNA of said genes in the biological sample. Using a DNA chip is particularly useful in that respect. The assay using such a chip is indeed reliable, fast, and cheap.

A further subject of the invention is the DNA chip that allows performing such method.

### Detailed description of the invention

The inventors examined the expression of a panel of genes involved in cell cycle progression, DNA repair, and apoptosis that may have a putative role in trastuzumab resistance, in a series of breast carcinomas that had been treated with trastuzumab-based neoadjuvant chemotherapy. In parallel, they used microarray analysis on the same tumor samples in order to identify a potential marker of pCR that may have prognostic value in the identifying patients who are more likely to respond to trastuzumab therapy.

On this basis, the inventors identified a set of genes whose combined expression profiles allow to distinguish patients between responder and non-responder to a treatment with a HER2 blocking agent, preferably an anti-HER2 antibody such as trastuzumab.

In practice, the rapid determination of the expression level of said genes, e.g. by a quantitative RT-PCR, offers a powerful tool for predicting responsiveness to a HER2 blocking agent, preferably an anti-HER2 antibody such as trastuzumab,.

The study presented in the Examples shows that such analysis allows to predict efficacy of a HER2 blocking agent, preferably an anti-HER2 antibody such as trastuzumab, with a sensitivity of 100%, a specificity of 78%, and a positive predictive value of 67%, and a negative predictive value of 100%.

### Patients

The term "patient" refers to any subject (preferably human) afflicted with a disease likely to benefit from a treatment with a HER2 blocking agent, in particular a HER2-related disease. The patient may be a man or a woman, preferably a woman, especially a woman with breast cancer.

Such diseases include benign and malignant tumors; leukemias and lymphoid malignancies; neuronal, glial, astrocytal, hypothalamic and other glandular, macrophagal, epithelial, stromal and blastocoelic disorders; and inflammatory, angiogenic and immunologic disorders. Cancers are more preferably targeted.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, liver cancer, prostate cancer, vulval cancer, thyroid cancer, hepatic carcinoma and various types of head and neck cancer.

The patient is preferably affected with a HER2-positive disease, in particular a HER2-positive cancer.

Preferably the HER2 positive cancer is primary tumor. Alternatively the HER2 positive cancer is a secondary tumor such as *e*.*g*. a locally advanced cancer or a metastatic cancer.

The term "HER2-positive" means that the HER2 protein is overexpressed, *i*.*e*. shows an abnormal level of expression in a cell from a disease within a specific tissue or organ of the patient relative to the level of expression in a normal cell from that tissue or organ. Patients having a cancer characterized by overexpression of the HER2 receptor can be determined by standard assays known in the art. Preferably overexpression is measured in fixed cells of frozen or paraffin-embedded tissue sections using immunohistochemical (IHC) detection. When coupled with histological staining, localization of the targeted protein can be determined and extent of its expression within a tumor can be measured both qualitatively and semi-quantitatively. Such IHC detection assays are known in the art and include the Clinical Trial Assay (CTA), the commercially available LabCorp® 4D5 test, and the commercially available DAKO HercepTest® (DAKO, Carpinteria, Calif.). The latter assay uses a specific range of 0 to 3+ cell staining (0 being normal expression, 3+ indicating the strongest positive expression) to identify cancers having overexpression of the HER2 protein. Thus, patients having a cancer characterized by overexpression of the HER2 protein in the range of 1+, 2+, or 3+, preferably 2+ or 3+, more preferably 3+ are particularly encompassed.

Using standard detection assays, several types of cancers have been characterized as having cells that overexpress the HER2 protein. Such cancers include, but are not limited to, breast, gastric, endometrial, salivary gland, non-small cell lung, pancreatic, renal, ovarian, peritoneal, prostate, bladder, colorectal cancers, and glioblastomas. Methods of the invention are useful in the treatment/management of any such cancer whose cells overexpress the HER2 protein. Of particular interest is breast cancer.

A "responder" patient, or group of patients, refers to a patient, or group of patients, who shows or will show a clinically significant relief in the disease when treated with a HER2 blocking agent.

The expression level of the genes or transcripts is determined and compared between a responder group and a non-responder group of patients. Said "expression level of genes or transcripts" corresponds to the combined expression profile of said genes or transcripts in either group.

The comparison between groups can be performed by computer tools, using the Mann and Whitney test performed with 1000 permutations (Zoe software, IGBM Strasbourg). These tools take into account the differential expression of the gene clusters, *i*.*e*. of the combination of the genes between groups, and generate an algorithm. The latter next allows for a prediction of the non-responder or responder status of any further patient, provided the same transcript level has been determined in said patient.

After being tested for responsiveness to a treatment with a HER2 blocking agent, the patients may be prescribed with a HER2 blocking agent with or without the same basic treatment.

While the methods of the invention are directed to treatment of an existing disease, such as cancer, it is recognized that the methods may be useful in preventing further disease development, including tumor outgrowths arising during therapy. The methods of the invention are particularly useful in the treatment of subjects having breast cancer, more particularly subjects having metastatic breast cancer and experiencing a relapse following one or more chemotherapy regimens for their metastatic disease.

### Chemotherapy choice

The method of the invention makes it possible to discriminate between "responder" and "non-responder" patients to a treatment with a HER2 blocking agent

"HER2 blocking agents" refer to molecules, such as proteins or small molecules, that can significantly reduce HER2 properties.

Such blocking agents include anti-HER2 antibodies, *e*.*g*. trastuzumab, pertuzumab, or cetuximab. Preferably the anti-HER2 antibody is trastuzumab.

Trastuzumab (sold under the tradename Herceptin®) is a recombinant humanized anti-HER2 monoclonal antibody used for the treatment of HER2 over- expressed/HER2 gene amplified metastatic breast cancer. Trastuzumab binds specifically to the same epitope of HER2 as the murine anti-HER2 antibody 4D5. Trastuzumab is a recombinant humanized version of the murine anti-HER2 antibody 4D5, referred to as rhuMAb 4D5 or trastuzumab) and has been clinically active in patients with HER2-overexpressing metastatic breast cancers that had received extensive prior anticancer therapy. Trastuzumab and its method of preparation are described in U.S. patent 5,821,337.

In a preferred embodiment, the method of the invention is useful for predicting whether a patient would be responsive to a treatment with a HER2 blocking agent combined with a taxane, such as docetaxel.

"Docetaxel" refers to the active ingredient of Taxotere®.

### The sets of predictive genes or transcripts

All the genes or transcripts identified are known per se, and listed in the below tables A, B and C.

Table A presents the set of four genes whose combined expression profile has been shown to be informative with regard to responsiveness to a treatment with HER2 blocking agent. These are the GPR22, PEX19, GRHL2 and DERL1 transcripts.

**TABLE A: subset of 4 genes (transcripts)**

| **Gene** | **GENBANK access number** | **Name** | **Seq ID NO** : |
|---|---|---|---|
| GPR22 | NM_005295 | G protein-coupled receptor 22 | 1 |
| PEX19 | NM_002857 | Peroxisomal biogenesis factor 19 | 3 |
| GRHL2 | NM_024915 | Grainyhead-like 2 (Drosophila) | 5 |
| DERL1 | NM_024295 | Derlin 1 | 7 |

In a particular embodiment, the method of the invention further comprises determining the expression level of the genes or transcripts of Table B, or of a subcombination thereof (combined with the set of four genes or transcripts as defined in Table A):

**TABLE B : Other transcripts of interest for the predictive method**

| **AgilentSpotID** | **GenbankID** | **UGCluster** | **Name** | **Symbol** | **p value** |
|---|---|---|---|---|---|
| as00595 | AK095652 | Hs.494822 | Hypothetical protein LOC158402 | LOC158402 | 0 |
| as02991 | NM_003390 | Hs.249441 | WEE1 homolog (S. pombe) | WEE1 | 0,001 |
| as03022 | AL117644 | Hs.429819 | Phosphatidylinositol transfer protein, alpha | PITPNA | 0,001 |
| as04760 | AK022035 | Hs.659665 | CDNA FLJ11973 fis, clone HEMBB1001221 | | 0 |
| as05104 | NM_002715 | Hs.483408 | Protein phosphatase 2 (formerly 2A), catalytic subunit, alpha isoform | PPP2CA | 0,001 |
| as06024 | NM_007145 | Hs.643436 | Zinc finger protein 146 | ZNF146 | 0,001 |
| as06108 | NM_020654 | Hs.529551 | SUMO1/sentrin specific peptidase 7 | SENP7 | 0 |
| as06408 | NM_080670 | Hs.406840 | Solute carrier family 35, member A4 | SLC35A4 | 0,001 |
| as06448 | XM_045127 | Hs.605380 | Homo sapiens KIAA1549 protein | KIAA1549 | 0 |
| as08524 | NM_003204 | Hs.514284 | Nuclear factor (erythroid-derived 2)-like 1 | NFE2L1 | 0,001 |
| as09257 | NM_005295 | Hs.657277 | G protein-coupled receptor 22 | GPR22 | 0 |
| as10291 | NM_006372 | Hs.571177 | Synaptotagmin binding, cytoplasmic RNA interacting protein | SYNCRIP | 0 |
| as11424 | NM_018691 | Hs.166551 | Chromosome 5 open reading frame 3 | C5orf3 | 0,001 |
| as11638 | NM_002857 | Hs.517232 | Peroxisomal biogenesis factor 19 | PEX19 | 0 |
| as12494 | NM_002558 | Hs.41735 | Purinergic receptor P2X, ligand-gated ion channel, 1 | P2RX1 | 0,001 |
| as15670 | NM_017964 | Hs.23248 | Solute carrier family 30 (zinc transporter), member 6 | SLC30A6 | 0 |
| as15820 | NM_003672 | Hs.127411 | CDC14 cell division cycle 14 homolog A (S. cerevisiae) | CDC14A | 0,001 |
| as16749 | NM_024915 | Hs.661088 | Grainyhead-like 2 (Drosophila) | GRHL2 | 0 |
| as18913 | NM_145204 | Hs.513002 | SUMO/sentrin specific peptidase family member 8 | SENP8 | 0,001 |
| as19535 | NM_002815 | Hs.655396 | Proteasome (prosome, macropain) 26S subunit, non-ATPase, 11 | PSMD11 | 0,001 |
| as20561 | NM_004937 | Hs.187667 | Cystinosis, nephropathic | CTNS | 0 |
| | NM_024295 | | | | |
| as21050 | | KIAA1549 | Derlin 1 | DERL1 | 0 |
| as21549 | NM_032816 | Hs.599703 | Coiled-coil domain containing 123 | CCDC123 | 0 |
| as21885 | NM_002730 | Hs.631630 | Protein kinase, cAMP-dependent, catalytic, alpha | PRKACA | 0,001 |
| as22719 | NM_000227 | Hs.436367 | Laminin, alpha 3 | LAMA3 | 0,001 |
| as22767 | NM_017694 | Hs.644886 | FLJ20160 protein | FLJ20160 | 0,001 |
| as24280 | XM_295178 | Not available | Homo sapiens hypothetical LOC340171 | LOC340171 | 0 |
| as25304 | NM_004603 | Hs.647024 | Syntaxin 1A (brain) | STX1A | 0,001 |

**TABLE C : Subgroup of transcripts of interest for the predictive method**

| **Gene** | **Genbank ID** | **Description** |
|---|---|---|
| LOC158402 | AK095652 | Hypothetical protein LOC158402 |
| | AK022035 | CDNA FLJ11973 fis, clone HEMBB1001221 |
| SENP7 | NM_020654 | SUMO1/sentrin specific peptidase 7 |
| KIAA1549 | XM_045127 | Homo sapiens KIAA1549 protein |
| GPR22 | NM_005295 | G protein-coupled receptor 22 |
| SYNCRIP | NM_006372 | Synaptotagmin binding, cytoplasmic RNA interacting protein |
| PEX19 | NM_002857 | Peroxisomal biogenesis factor 19 |
| SLC30A6 | NM_017964 | Solute carrier family 30 (zinc transporter), member 6 |
| GRHL2 | NM_024915 | Grainyhead-like 2 (Drosophila) |
| CTNS | NM_004937 | Cystinosis, nephropathic |
| DERL1 | NM_024295 | Derlin 1 |
| | | |
| CCDC123 | NM_032816 | Coiled-coil domain containing 123 |
| LOC340171 | XM_295178 | Homo sapiens hypothetical LOC340171 |

Among the 28 genes of Table B, 12 were shown to be more highly expressed in pCR tumor samples (WEE1, ZNF146, SENP7, GPR22, SYNCRIP, SLC30A6, GRHL2, CCDC123, STX1A, cDNA FLJ11973 fis, clone HEMBB1001221, KIAA1549 and Homo sapiens hypothetical LOC340171), and 16 genes were shown to be highly expressed in non-pCR samples (LOC158402, PITPNA, PPP2CA, SLC35A4, NFE2L1, C5orf3, PEX19, P2RX1, CDC14A, SENP8, PSMD11, CTNS, PRKACA, LAMA3, FLJ20160, and DERL1).

### Determination of expression level

Determination of the expression level of a gene or transcript can be performed by a variety of techniques, from a biological sample. The term "biological sample" means any biological sample derived from a patient, preferably a sample which contains nucleic acids. Examples of such samples include fluids, tissues, cell samples, organs, biopsies, etc. Most preferred samples are disease tissue samples. Blood, plasma, saliva, urine, seminal fluid, etc, may also be used. The biological sample may be treated prior to its use, *e*.*g*. in order to render nucleic acids available. Techniques of cell or protein lysis, concentration or dilution of nucleic acids, are known by the skilled person.

Generally, the expression level as determined is a relative expression level.

More preferably, the determination comprises contacting the sample with selective reagents such as probes, primers or ligands, and thereby detecting the presence, or measuring the amount, of polypeptide or nucleic acids of interest originally in the sample. Contacting may be performed in any suitable device, such as a plate, microtiter dish, test tube, well, glass, column, and so forth In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a preferred embodiment, the expression level may be determined by determining the quantity of mRNA.

Methods for determining the quantity of mRNA are well known in the art. For example the nucleic acid contained in the samples (*e*.*g*., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (*e*.*g*., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, *e*.*g*., 50 % formamide, 5x or 6x SCC. SCC is a 0.15 M NaCl, 0.015 M Na-citrate).

The nucleic acid primers or probes used herein may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, *e*.*g*. by using radioactive or fluorescent labelling. Many variants of the microarray hybridization technology are available to the man skilled in the art (see e.g. the review by Hoheisel, et 2006)

In this context, the invention further provides a DNA chip comprising a solid support which carries nucleic acids that are specific to GPR22, PEX19, GRHL2 and DERL1 genes.

In a particular embodiment, the invention provides a DNA chip which further carries nucleic acids that are specific to any or all of the transcripts listed in Table B, or a subcombination thereof.

In a preferred embodiment, the invention more particularly provides a DNA chip comprising a solid support which carries nucleic acids that are specific to GPR22, PEX19, GRHL2 and DERL1 genes, and which further carries nucleic acids that are specific to any or all of the transcripts listed in Table C.

Other methods for determining the expression level of said genes include the determination of the quantity of proteins encoded by said genes.

Such methods comprise contacting a biological sample with a binding partner capable of selectively interacting with a marker protein present in the sample. The binding partner is generally an antibody, that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (*e*. *g*., in membrane or microtiter well form); polyvinylchloride (*e*. *g*., sheets or microtiter wells); polystyrene latex (*e*.*g*., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

### Therapeutic applications

A method for treating a HER2 -related disease is contemplated, which method comprises
a) a preliminary step of testing whether a patient with a HER2 - related disease would be responsive to a treatment with a HER2 blocking agent, by determining the expression level of four genes in a biological sample of said patient, wherein said genes are GPR22, PEX19, GRHL2 and DERL1; hereby classifying the patient as responder or non responder;
b) a step of administering a HER2 blocking agent to a patient classified as responder.

The classification of the patient -as responder or non-responder- is made according to the combined expression level of said four genes. It allows to define a subgroup of patients who will be responsive to a treatment with a HER2 blocking agent.

Another particular subject of the invention is thus a HER2 blocking agent, such as an anti-HER2 antibody, for treating a patient with a HER2-related disease, such as a cancer, and classified as responder to a treatment with a HER2 blocking agent, by the method described above.

The example illustrates the invention without limiting its scope.

### EXAMPLE 1: GENE EXPRESSION PROFILE AND RESPONSE TO TRASTUZUMAB-DOCETAXEL-BASED TREATMENT IN BREAST CARCINOMA

### Materials and Methods

### Patients and samples

The inventors retrospectively studied a population of 38 patients who had received trastuzumab in combination with chemotherapy as primary systemic therapy for their operable, HER2-positive, stage II/III breast cancer (Table 1).

**Table 1: Demographic data**

| | | **Training set** | **Independent set** | **Total** |
|---|---|---|---|---|
| | | **(n=25)** | **(n=13)** | **(n=38)** |
| **Age (years)** | | | | |
| | ≤50 | 13 | 9 | 22 |
| | >50 | 12 | 4 | 16 |
| **SBR grade** | | | | |
| | I | 1 | 0 | 1 |
| | II | 14 | 8 | 22 |
| | III | 10 | 4 | 14 |
| | Unknown | 0 | 1 | 1 |
| **Hormone receptors** | | | | |
| | ER-negative | 13 | 3 | 16 |
| | ER-positive | 12 | 10 | 22 |
| | PR-negative | 16 | 3 | 19 |
| | PR-positive | 9 | 10 | 19 |
| **Tumor size (cm)** | | | | |
| | <2 | 1 | 0 | 1 |
| | 2-4 | 17 | 12 | 29 |
| | >4 | 6 | 1 | 7 |
| | ND | 1 | 0 | 1 |
| **Treatment** | | | | |
| | TH | 18 | 11 | 29 |
| | TCH | 7 | 2 | 9 |
| **Pathological response** | | | | |
| | pCR | 11 | 4 | 15 |
| | non-pCR | 14 | 9 | 23 |

| | | | | |
|---|---|---|---|---|
| ER, estrogen receptor; non-pCR, absence of pathological complete response; ND, not determined; pCR, pathological complete response; PR, progesterone receptor SBR, Scarff-Bloom-Richardson; TCH, trastuzumab + carboplatin + docetaxel; TH, trastuzumab + docetaxel | | | | |

All patients provided written, informed consent for their tissue material and clinical data to be used for research purposes. Patients were treated in two open-label phase II clinical trials: TAXHER01 (n=29) and GETNA01 (n=10) (Coudert et al, 2006; Coudert et al, 2007).

All patients received weekly neoadjuvant trastuzumab (4 mg/kg loading dose followed by 2 mg/kg once weekly) in combination with either docetaxel alone (100 mg/m2 every 3 weeks for six cycles) or docetaxel (75 mg/m2 every 3 weeks for six cycles) combined with carboplatin (AUC 6) every 3 weeks for six cycles. pCR rates were assessed using Chevallier's classification (Chevallier et al, 1993) 3 weeks after the last course of trastuzumab-containing neoadjuvant treatment. An absence of disease in the breast or in the lymph nodes, with or without in situ carcinoma, was considered to be a pCR. HER2 status was determined using both immunohistochemistry and fluorescence in situ hybridization (Arnould et al, 2007).

Needle core biopsies were taken at baseline, with one used for the initial diagnosis and two used for RNA extraction. All tissue samples were snap frozen and stored in liquid nitrogen, and only samples containing ≥30% tumor cells were analyzed further.

### RNA extraction

Total RNA was extracted from tissue samples by using the TRlzol® method as recommended by the manufacturer (Invitrogen Corporation, Carlsbad, CA, USA). The quantity and purity of the extracted RNA were assessed using a NanoDrop® 1000 spectrophotometer (NanoDrop, Wilmington, DE, USA) at 260 and 280 nm (the A260/280 ratio of pure RNA is higher than 1.8). The quality of the extracted RNA was determined using an Agilent 2100 bioanalyzer (Agilent, Santa Clara, CA, USA). Total RNA from a pool of four normal mammary tissues was used as a normal sample, and RNA extracted from the MCF-7 human breast cancer cell line was used to calibrate real-time quantitative and reverse transcriptase polymerase chain reaction (RT-PCR).

### RT-PCR and real-time quantitative PCR

One microgram of total RNA was reverse transcribed in 20 µl of RT-PCR (Arnal et al, 2000). The real-time quantitative PCR was performed on ABI PRISM® 7300 (Applied Biosystems, Foster City, CA, USA) using the TaqMan® method. Analysis of 18S ribosomal RNA was used to assess complementary DNA (cDNA) quality and as a reference control. Results were analyzed at the Ct level and references for the genes analyzed are summarized in Table 2.

**Table 2: References and nucleotide sequences of primers and probes used in this study**

| **Function** | **Gene or transcript** | **Reference NCBI** | **Reference or sequences** |
|---|---|---|---|
| **Cell cycle** | *cdc27* | NM_001256 | Hs01559427_m1 |
| | *SKP2* | NM_032637 | Hs01021867_m1 |
| | *p27* | NM_004064 | Hs00153277_m1 |
| | *p53* | NM_000546 | Hs00153340_m1 |
| | *c-Myc* | NM_002467 | Hs00153408_m1 |
| | *Cyclin B2* | NM_004701 | Hs00270424_m1 |
| | *RBX1* | NM_014248 | Hs00360274_m1 |
| | *CCL4* | NM_002984 | Hs99999148_m1 |
| | *CDC45l* | NM_003504 | Hs00907337_m1 |
| | *XRCC2* | NM_005431 | Hs00538799_m1 |
| | *ERCC2* | NM_000400 | Hs00361161_m1 |
| **DNA repair** | *MREIIA* | NM_005591 | Hs00967442_m1 |
| | *HMOX2* | NM_002134 | Hs01558390_m1 |
| | *MSH5* | NM_002441 | Hs00159268_m1 |
| | | | F: 5'-ccagatgacgaccccatagag-3' (SEQ ID NO:9) |
| **Apoptosis** | *Survivin* | NM_001168 | R: 5'-ttgttggtttcctttgcaatttt-3'(SEQ ID NO:10) |
| | | | P: 5'-cattcgtccggttgcgctttcc-3' (SEQ ID NO:11) |
| | | | F: 5'-aagaactggcccttcttgga-3'(SEQ ID NO:12) |
| | *Survivin-2B* | NM_001012271 | R: 5'-ccaagtgctggtattacaggcgta-3' (SEQ ID NO:13) |
| | | | P: 5'-actgccccactgagaacgagcca-3'(SEQ ID NO:14) |
| | | | F:5'-cccagtgtttcttctgcttcaa-3' (SEQ ID NO:15) |
| | *Survivin*-Δ*Ex3* | NM_001012270 | R: 5'-ttcttcgcagtttcctcaaattct-3' (SEQ ID NO:16) |
| | | | P: 5'-acgaccccatgcaaaggaaaccaaca-3' (SEQ ID NO:17) |
| | | | F: 5'-ccagatgacgaccccatagag-3' (SEQ ID NO:18) |
| | *Survivin-3B* | AB154416 | R: 5'-aagaactggcccttcttgga-3' (SEQ ID NO:19) P: 5'-cattcgtccggttgcgctttcc-3' (SEQ ID NO:20) |
| | | | F: 5'-gctttgttttgaactgagttgtcaa-3'(SEQ ID NO:21) |
| | *Survivin-2*α | | R: 5'-gcaatgagggtggaaagca-3' (SEQ ID NO:22) |
| | | | P: 5'-agatttgagttgcaaagacacttagtatgggaggg-3' (SEQ ID NO:23) |
| | | | F: 5'-ctggactgtggcattgagaca-3' (SEQ ID NO:24) |
| | *Caspase-3* | NM_032991 | R: 5'-agtcggcctccatggtattt-3' (SEQ ID NO:25) |
| | | | P: 5'-tggtgttgatgatgacatggcgtgtc-3' (SEQ ID NO:26) |
| | | | F:5'-agaagtctaactggaaaacccaaact-3' (SEQ ID NO:27) |
| | *Caspase-3s* | | R: 5'-caaagcgactggatgaacca-3' (SEQ ID NO:28) |
| | | | P: 5'-attattcaggttattattcttggcg-3' (SEQ ID NO:29) |
| | *Casp8AP2* | NM_012115 | Hs00201640_m1 |
| | *Caspase-9* | NM_032996 | Hs00154261_m1 |
| | *ASC* | NM_013258 | Hs0154724_gH |
| | *Fasl* | NM_000639 | Hs00899442_m1 |
| | *LTBR* | NM_002342 | Hs00158922_m1 |
| | *HSP90* | NM_001040141 | Hs00743767_sH |
| | *TRAF5* | NM_004619 | Hs01072220_m1 |
| | *BCL-x* | NM_001191 | Hs00236329_m1 |
| | *CD40* | NM_000074 | Hs99999100_s1 |
| **Housekeeping** | 18S | x03205.1 | Hs99999901_s1 |

| | | | |
|---|---|---|---|
| F, forward; NCBI, National Center for Biotechnology Information; P, probe; R, reverse | | | |

Survivin, caspase-3, and their splice variant expressions were determined by design primers and probes labeled at the 5' end with FAM and at the 3' end with TAMRA. Assays on Demand (Applied Biosystems) were used for the other studied genes. Amplifications were performed in a total volume of 25 µl in the presence of 12.5 µl Universal Master Mix (Applied Biosystems), 150 nM of each primer and 200 nM probe for survivin, 300 nM primers and 150 nM probes for survivin-DEx3, 300 nM primers and 200 nM probe for survivin-2B, 300 nM primers and 150 nM probe for survivin-3B, 600 nM primers and 200 nM probe for survivin-2a, caspase-3, and their splice variants caspase-3s, or 1.25 µl of Assays on Demand and 12 ng cDNA (or water as a negative control). The PCR program consisted of a 10-min initial denaturation step at 95°C, followed by 40 cycles of 15 sec at 95°C, and 1 min at 60°C. cDNA from MCF-7 cells was analyzed simultaneously as a control and all samples were amplified in duplicate, with results analyzed using either the 2-DCt method for expression comparison or the 2-DDCt method (Livak et al, 2001) for statistical analysis.

The Mann-Whitney U and Chi2 tests were used to compare gene expression with pathological response, with a threshold corresponding to normal mammary tissue relative expression value used to separate the population into two groups. Statistical significance was considered when p<0.05.

### Microarray experiment

Microarray analyses were performed using the Affymetrix-Microarray Platform of the Institute of Genetics and Molecular and Cellular Biology (IGBMC) and Génopole Alsace-Lorraine (Dr Philippe Kastner). The analysis used samples from 25 patients (11 with pCR and 14 with non-pCR) and the resulting profile was validated using an independent and blinded group of 13 patients (four with pCR and nine with non-pCR).

The fluorescent nucleic acids hybridized onto the microarrays were prepared from total RNA. One microgram of total RNA was reverse transcribed into cDNA using a poly-dT with an extended region as a 3' end primer. After second-strand synthesis, all the different double-strand cDNAs had a common 3' end extension, which was used as a specific annealing site during PCR amplification. This unidirectional PCR amplification produced single-strand linear PCR products, which were labeled by random priming with dUTP-Cy5 (red) for the test samples or with dUTP-Cy3 (green) for the reference samples. Test and reference samples were co-hybridized onto microarrays. Human microarrays from the Affymetrix-Microarray Platform of the IGBMC and Génopole Alsace-Lorraine were used, onto which 25,000 genes were spotted. Reference genes were eliminated. Hybridized slides were scanned to detect fluorescence signals at high resolution. Fluorescent intensities were normalized and standardized by using the IGBMC in-house 'Elea' software followed by a LOWESS (LOcal Weighted Estimates of Smooth Scatterplots) fitting-based method. Briefly, genes were selected as invariants from ranks of values in the Cy3 and Cy5 channels, and were then used in the LOWESS algorithm to compute the normalization factor between the two channel values. This generated two values: the signal value A = Log2 (test value * reference value)/2; and the log ratio M = Log2 (test value / reference value).

### Microarray data analysis

Using the A values, the inventors determined the lowest median expression level of the population and excluded every gene with an A value lower than this. Using this heuristic filtering, we identified 14,829 genes to analyze further. From this subset of genes, statistical filtering was performed on the M values using IGBMC in-house statistical 'Zoe' software. The Mann-Whitney U test was then used with 1000 permutations to compare pCR and non-pCR rates, with p<0.002 considered significant.

### Results

### Analysis of selected gene expression by quantitative RT-PCR

When the relative expression of genes associated with cell cycle progression was compared with pathological response, it was found that the expression of these genes did not correlate with the observed pathological response, as shown by the Chi2 test. The inventors next compared the relative expression of DNA repair genes with pathological response, and the results similarly showed that the expression of these genes did not correlate with pathological response to a trastuzumab-docetaxel-based regimen. No relationship was also found between the relative expression of apoptotic genes and pathological responses. However, the Chi2 test did show a significant (p<0.0001) inverse relationship between expression of bcl-xL and response to trastuzumab-docetaxel-based treatment.

### Microarray data analysis

Of the 25 patients in the training set, 11 (44%) patients showed pCR and 14 (56%) had non-pCR. Microarray analysis of tumor samples from these patients indicated that expression significantly differed between pCR and non-pCR tumor samples for 28 genes. Among these 28 genes, 12 were more highly expressed in pCR tumor samples (WEE1, ZNF146, SENP7, GPR22, SYNCRIP, SLC30A6, GRHL2, CCDC123, STX1A, cDNA FLJ11973 fis, clone HEMBB1001221, KIAA1549 and Homo sapiens hypothetical LOC340171), and 16 genes were highly expressed in non-pCR samples (LOC158402, PITPNA, PPP2CA, SLC35A4, NFE2L1, C5orf3, PEX19, P2RX1, CDC14A, SENP8, PSMD11, CTNS, PRKACA, LAMA3, FLJ20160, and DERL1 (see Table B). In addition, there was no difference observed for treatment effect (TAXHER01 or GETNA01) on this 28-gene expression profile.

The discriminatory 28-gene profile was then validated using the independent cohort of 13 patients. The analysis of the profile was performed without prior knowledge of the patients' pathological response. Each patient's tumor sample was classified using a correlation coefficient based on the mean expression value of each selected gene for the pCR and non-pCR subsets. A patient was classified as being in the pCR group when their correlation coefficient was higher, with mean values above the non-pCR values, and vice versa. Using this approach, the present 28-gene profile correctly classified the four pCR patients as having the pCR expression profile, and 8/9 non-pCR patients into the non-pCR profile. Thus, the present 28-gene profile for a trastuzumab-docetaxel-based regimen exhibited 100% sensibility, 89% specificity, and 92% accuracy (Table 4).

**Table 4: Performance of the 28-gene (Table B) expression profile for the independent cohort response prediction**

| | | **Predicted** | | |
|---|---|---|---|---|
| | | **pCR** | **Non-pCR** | **Total** |
| **Observed** | **pCR** | 4 | 0 | 4 |
| | **Non-pCR** | 1 | 8 | 9 |
| | **Total** | 5 | 8 | 13 |

| | | **Cases** | **Percentage** | |
|---|---|---|---|---|
| | **Sensitivity** | 4/4 | 100 | |
| | **Specificity** | 8/9 | 89 | |
| | **Positive prediction value** | 4/5 | 80 | |
| | **Negative prediction value** | 8/8 | 100 | |
| | **Accuracy** | 12/13 | 92 | |

| | | | | |
|---|---|---|---|---|
| Non-pCR, non-pathological complete response; pCR, pathological complete response | | | | |

### Conclusion

Using microarray analysis, the inventors generated a 28-gene profile that can discriminate between tumor samples that would attain a pCR and those that would not in response to treatment with a trastuzumab-docetaxel-based regimen, with 92% accuracy. This profile was not affected by treatment effect (TAXHER01 or GETNA01), and the results confirm previous analyses from these two studies that have commented on the association between pCR and HER2 amplification (Arnould et al, 2007).

Importantly, the present results also teach that genes not involved in classical cancer pathways such as apoptosis, cell cycle progression, or DNA repair are involved in determining responses to a trastuzumab-docetaxel-based regimen.

### EXAMPLE 2: DEFINITIONS OF SUBGROUPS OF GENES

Using the same method as the one described in Example 1, the inventors showed that subgroups of genes were discriminative too.

**Table 5: Performance of the expression profile of genes of Table C-subgroup for the independent cohort response prediction**

| | | **Predicted** | | |
|---|---|---|---|---|
| | | **pCR** | **Non-pCR** | **Total** |
| **Observed** | **pCR** | 4 | 0 | 4 |
| | **Non-pCR** | 2 | 7 | 9 |
| | **Total** | 6 | 7 | 13 |

| | | **Cases** | **Percentage** | |
|---|---|---|---|---|
| | **Sensitivity** | 4/4 | 100 | |
| | **Specificity** | 7/9 | 78 | |
| | **Positive prediction value** | 4/6 | 67 | |
| | **Negative prediction value** | 7/7 | 100 | |
| | **Accuracy** | 11/13 | 85 | |

**Table 6: Performance of the expression profile of genes of Table A-subgroup for the independent cohort response prediction**

| | | **Predicted** | | |
|---|---|---|---|---|
| | | **pCR** | **Non-pCR** | **Total** |
| **Observed** | **pCR** | 4 | 0 | 4 |
| | **Non-pCR** | 2 | 7 | 9 |
| | **Total** | 6 | 7 | 13 |

| | | **Cases** | **Percentage** | |
|---|---|---|---|---|
| | **Sensitivity** | 4/4 | 100 | |
| | **Specificity** | 7/9 | 78 | |
| | **Positive prediction value** | 4/6 | 67 | |
| | **Negative prediction value** | 7/7 | 100 | |
| | **Accuracy** | 11/13 | 85 | |

### REFERENCES

- Arnal M, Franco N , Fargeot P, Riedinger JM, Brunet-Lecomte P, Lizard-Nacol S. Enhancement of mdr1 gene expression in normal tissue adjacent to advanced breast cancer. Breast Cancer Res Treat 2000;61:13-20.
- Arnould L, Arveux P, Couturier J, Gelly-Marty M, Loustalot C, Ettore F et al. Pathologic complete response to trastuzumab-based neoadjuvant therapy is related to the level of HER-2 amplification. Clin Cancer Res 2007;13:6404-9.
- Buzdar AU, Ibrahim NK, Francis D, Booser DJ, Thomas ES, Theriault RL et al. Significantly higher pathologic complete remission rate after neoadjuvant therapy with trastuzumab, paclitaxel, and epirubicin chemotherapy: results of a randomized trial in human epidermal growth factor receptor 2-positive operable breast cancer. J Clin Oncol 2005;23:3676-85.
- Chevallier B, Roche H, Olivier JP, Chollet P, Hurteloup P. Inflammatory breast cancer. Pilot study of intensive induction chemotherapy (FEC-HD) results in a high histologic response rate. Am J Clin Oncol 1993;16:223-8.
- Cobleigh MA, Vogel CL, Tripathy D, Robert NJ, Scholl S, Fehrenbacher L et al. Multinational study of the efficacy and safety of humanized anti-HER2 monoclonal antibody in women who have HER2-overexpressing metastatic breast cancer that has progressed after chemotherapy for metastatic disease. J Clin Oncol 1999;17:2639-48.
- Coudert BP, Arnould L, Moreau L, Chollet P, Weber B, Vanlemmens L et al. Pre-operative systemic (neo-adjuvant) therapy with trastuzumab and docetaxel for HER2-overexpressing stage II or III breast cancer: results of a multicenter phase II trial. Ann Oncol 2006;17:409-14.
- Coudert BP, Largillier R, Arnould L, Chollet P, Campone M, Coeffic D et al. Multicenter phase II trial of neoadjuvant therapy with trastuzumab, docetaxel, and carboplatin for human epidermal growth factor receptor-2-overexpressing stage II or III breast cancer: results of the GETN(A)-1 trial. J Clin Oncol 2007;25:2678-84.
- Harris LN, You F, Schnitt SJ, Witkiewicz A, Lu X, Sgroi D et al. Predictors of resistance to preoperative trastuzumab and vinorelbine for HER2-positive early breast cancer. Clin Cancer Res 2007;13:1198-207.
- Hoheisel, Nat Rev Genet. 2006 Mar;7(3):200-10
- Livak KJ, Schmittgen TD. Analysis of relative gene expression data using real-time quantitative PCR and the 2(-Delta Delta C(T)) Method. Methods 2001;25:402-8.

## Claims

1. An *in vitro* method for predicting whether a patient would be responsive to a treatment with an anti-HER2 antibody, which method comprises determining the expression level of at least 4 genes in a biological sample of said patient, wherein said genes are GPR22, PEX19, GRHL2 and DERL1.

2. The method of claim 1, wherein the patient is affected with a HER2-positive cancer.

3. The method of claim 1 or 2, wherein the patient is with breast cancer.

4. The method of any of claims 1 to 3, wherein the HER2 blocking agent is an anti-HER2 antibody

5. The method of claim 4, wherein the anti-HER2 antibody is trastuzumab.

6. The method of any of claims 1 to 5, for predicting whether a patient would be responsive to a treatment with a HER2 blocking agent, combined with a taxane, such as docetaxel.

7. The method of any of claims 1 to 6, further comprising the step of comparing the combined expression level of said genes with reference values obtained from responder and non-responder groups of patients.

8. The method of any of claims 1 to 7, wherein the biological sample is a disease tissue sample.

9. The method of any of claims 1 to 8, wherein the expression level is determined by quantifying the level of mRNA of said genes in the biological sample.

10. The method of claim 9, wherein the expression level is determined by real-time quantitative or semi-quantitative RT-PCR.

11. The method of claim 9 or 10, wherein the expression level is determined by using a DNA chip.

12. The method of any of claims 1 to 11, further comprising determining the expression level of the transcripts listed in Table B, or of a subcombination thereof.

13. The method of claim 12, further comprising determining the expression level of any or all of the transcripts listed in Table C.

14. A DNA chip comprising a solid support which carries nucleic acids that are specific to GPR22, PEX19, GRHL2 and DERL1 genes.

15. The chip of claim 14, which further carries nucleic acids that are specific to any or all of the transcripts listed in Table B, or a subcombination thereof.

16. The chip of claim 15, which further carries nucleic acids that are specific to any or all of the transcripts listed in Table C.

17. A HER2 blocking agent, such as an anti-HER2 antibody, for treating a patient with a HER2-related disease, such as a cancer, and classified as responder to a treatment with a HER2 blocking agent, by the method of any of claims 1 to 13.


SEQUENCE LISTING

<110>  Centre Georges François Leclerc

<120>  A method for predicting responsiveness to a treatment with an
       anti-HER2 antibody

<130>  B733EP00

<160>  29

<170>  PatentIn version 3.3

<210>  1
<211>  2976
<212>  DNA
<213>  Homo sapiens


<220>
<221>  CDS
<222>  (1347)..(2648)
<223>  GPR22

<400>  1
gtcattctgc aaaagctgcc cgaacaggct ttcctctgaa aagcagtgcc gttccttttt      60

agaaagactg aatctaaatg ttgtctctgg agacaagaag ccttcagtat gttaaattac     120

tttcattatg tattttcaga tgcttattga ttccacagta ggaagagtga gagactgcag     180

cagcctctaa gcagcactac atgttccata cattagcagt actgctgaaa caatggcaca     240

ctacagacac atattttcat taaggtcatt ttcgaagaga tgtttatgac cctcttcccc     300

cagtcctcta ctaacaagtg aacaaaatga atcaatccaa actggaaatg cttcaactac     360

atcaagaatt tatcaaatct ttagcagagg gccaatagca gctaattcag tatttacaac     420

tgacaatatg aagaatgcaa ttgactgagc atctccctag ctgtctgaac tacgaactgc     480

aagatgttct tgtaacacga ctttaagaca ttaaggagtt aaaaccaggg aataggtcta     540

cattactgat ggaatataaa aaatcaactg tatcctaaga agatgctaca taaaataacc     600

acaaaaagaa aaacaatata actgtaaaag cctgaaaaga atttttaaaa ggggaagttt     660

atactttcat ataccagaat tgtggaagtt actgattctg gaagacataa tgaaacatga     720

atttccaaaa agaaaagaaa atactttatc agcacacaaa aggaagattt aggaagtgtt     780

ttctgcacta aatattcaga tattcatatc aattggtatg actaatggat ttttctatct     840

gacttttatg accaattatg tatcctcttc taatgaaaac aaacaaaatt aaacagcaga     900

tggtttttat caaaaggaca tggcctggat ttataatata aagcaagtta tgtgatcaag     960

aaatcatttc aaaatagtga gcactgctat taaaaacaga tttacaatgg taacaaaagg    1020

atgtctaaat atattttaga agctacaaac gttatgtttc cttttttgtt ttcacatatt    1080

ctggaaaata aagaaatatt atcatgtact ccatcaaagg gaaacataat tcctatcatc 1140

tgaggaaatt cctcttggcc gtgacttttt aaagcaaaac aaatacaaat attatgtact 1200

gttctttaga aatccatcag ccaactaaat ctcataatgc atgcagttga agtattggag 1260

agaaaacgaa agaattccta caagacatga aataaaacac agctacttca ctgttgtcag 1320

ggaaaaaaac caactgctcc aaaaga atg tgt ttt tct ccc att ctg gaa atc 1373
Met Cys Phe Ser Pro Ile Leu Glu Ile
1               5

aac atg cag tct gaa tct aac att aca gtg cga gat gac att gat gac 1421
Asn Met Gln Ser Glu Ser Asn Ile Thr Val Arg Asp Asp Ile Asp Asp
10                  15                  20                  25

atc aac acc aat atg tac caa cca cta tca tat ccg tta agc ttt caa 1469
Ile Asn Thr Asn Met Tyr Gln Pro Leu Ser Tyr Pro Leu Ser Phe Gln
                30                  35                  40

gtg tct ctc acc gga ttt ctt atg tta gaa att gtg ttg gga ctt ggc 1517
Val Ser Leu Thr Gly Phe Leu Met Leu Glu Ile Val Leu Gly Leu Gly
            45                  50                  55

agc aac ctc act gta ttg gta ctt tac tgc atg aaa tcc aac tta atc 1565
Ser Asn Leu Thr Val Leu Val Leu Tyr Cys Met Lys Ser Asn Leu Ile
        60                  65                  70

aac tct gtc agt aac att att aca atg aat ctt cat gta ctt gat gta 1613
Asn Ser Val Ser Asn Ile Ile Thr Met Asn Leu His Val Leu Asp Val
    75                  80                  85

ata att tgt gtg gga tgt att cct cta act ata gtt atc ctt ctg ctt 1661
Ile Ile Cys Val Gly Cys Ile Pro Leu Thr Ile Val Ile Leu Leu Leu
90                  95                  100                 105

tca ctg gag agt aac act gct ctc att tgc tgt ttc cat gag gct tgt 1709
Ser Leu Glu Ser Asn Thr Ala Leu Ile Cys Cys Phe His Glu Ala Cys
                110                 115                 120

gta tct ttt gca agt gtc tca aca gca atc aac gtt ttt gct atc act 1757
Val Ser Phe Ala Ser Val Ser Thr Ala Ile Asn Val Phe Ala Ile Thr
            125                 130                 135

ttg gac aga tat gac atc tct gta aaa cct gca aac cga att ctg aca 1805
Leu Asp Arg Tyr Asp Ile Ser Val Lys Pro Ala Asn Arg Ile Leu Thr
        140                 145                 150

atg ggc aga gct gta atg tta atg ata tcc att tgg att ttt tct ttt 1853
Met Gly Arg Ala Val Met Leu Met Ile Ser Ile Trp Ile Phe Ser Phe
    155                 160                 165

ttc tct ttc ctg att cct ttt att gag gta aat ttt ttc agt ctt caa 1901
Phe Ser Phe Leu Ile Pro Phe Ile Glu Val Asn Phe Phe Ser Leu Gln
170                 175                 180                 185

agt gga aat acc tgg gaa aac aag aca ctt tta tgt gtc agt aca aat 1949
Ser Gly Asn Thr Trp Glu Asn Lys Thr Leu Leu Cys Val Ser Thr Asn
                190                 195                 200

gaa tac tac act gaa ctg gga atg tat tat cac ctg tta gta cag atc 1997

Glu Tyr Tyr Thr Glu Leu Gly Met Tyr Tyr His Leu Leu Val Gln Ile
            205                 210                 215

cca ata ttc ttt ttc act gtt gta gta atg tta atc aca tac acc aaa     2045
Pro Ile Phe Phe Phe Thr Val Val Val Met Leu Ile Thr Tyr Thr Lys
        220                 225                 230

ata ctt cag gct ctt aat att cga ata ggc aca aga ttt tca aca ggg     2093
Ile Leu Gln Ala Leu Asn Ile Arg Ile Gly Thr Arg Phe Ser Thr Gly
    235                 240                 245

cag aag aag aaa gca aga aag aaa aag aca att tct cta acc aca caa     2141
Gln Lys Lys Lys Ala Arg Lys Lys Lys Thr Ile Ser Leu Thr Thr Gln
250                 255                 260                 265

cat gag gct aca gac atg tca caa agc agt ggt ggg aga aat gta gtc     2189
His Glu Ala Thr Asp Met Ser Gln Ser Ser Gly Gly Arg Asn Val Val
            270                 275                 280

ttt ggt gta aga act tca gtt tct gta ata att gcc ctc cgg cga gct     2237
Phe Gly Val Arg Thr Ser Val Ser Val Ile Ile Ala Leu Arg Arg Ala
            285                 290                 295

gtg aaa cga cac cgt gaa cga cga gaa aga caa aag aga gtc ttc agg     2285
Val Lys Arg His Arg Glu Arg Arg Glu Arg Gln Lys Arg Val Phe Arg
        300                 305                 310

atg tct tta ttg att att tct aca ttt ctt ctc tgc tgg aca cca att     2333
Met Ser Leu Leu Ile Ile Ser Thr Phe Leu Leu Cys Trp Thr Pro Ile
    315                 320                 325

tct gtt tta aat acc acc att tta tgt tta ggc cca agt gac ctt tta     2381
Ser Val Leu Asn Thr Thr Ile Leu Cys Leu Gly Pro Ser Asp Leu Leu
330                 335                 340                 345

gta aaa tta aga ttg tgt ttt tta gtc atg gct tat gga aca act ata     2429
Val Lys Leu Arg Leu Cys Phe Leu Val Met Ala Tyr Gly Thr Thr Ile
                350                 355                 360

ttt cac cct cta tta tat gca ttc act aga caa aaa ttt caa aag gtc     2477
Phe His Pro Leu Leu Tyr Ala Phe Thr Arg Gln Lys Phe Gln Lys Val
            365                 370                 375

ttg aaa agt aaa atg aaa aag cga gtt gtt tct ata gta gaa gct gat     2525
Leu Lys Ser Lys Met Lys Lys Arg Val Val Ser Ile Val Glu Ala Asp
        380                 385                 390

ccc ctg cct aat aat gct gta ata cac aac tct tgg ata gat cct aaa     2573
Pro Leu Pro Asn Asn Ala Val Ile His Asn Ser Trp Ile Asp Pro Lys
    395                 400                 405

aga aac aaa aaa att acc ttt gaa gat agt gaa ata aga gaa aaa tgt     2621
Arg Asn Lys Lys Ile Thr Phe Glu Asp Ser Glu Ile Arg Glu Lys Cys
410                 415                 420                 425

tta gtg cct cag gtt gtc aca gac tag agaaaagtct cagtttcacc           2668
Leu Val Pro Gln Val Val Thr Asp
                430

aaatccacat tcaaatgagt tttaaattta aattgtaaaa actgatatta ctgccaaata   2728

taagaaaaat attttaagta ttggttatgt tgtaaatttt caatgtgaat gtcaattaga 2788

taggtcatat atattcaatt tcttcattac ttaatgtatt tgttgcatgg cagtttgtta 2848

aagtactatc atgtgtatat tttgtcaata ttatgtccaa cagaaaatat tcatgtaagt 2908

catatttttt aaggaataaa tacatagcct taaaacagtg tataacttta aaatgtaaaa 2968

aaaaaaaa 2976

<210> 2
<211> 433
<212> PRT
<213> Homo sapiens

<400> 2

Met Cys Phe Ser Pro Ile Leu Glu Ile Asn Met Gln Ser Glu Ser Asn
1               5                   10                  15

Ile Thr Val Arg Asp Asp Ile Asp Asp Ile Asn Thr Asn Met Tyr Gln
            20                  25                  30

Pro Leu Ser Tyr Pro Leu Ser Phe Gln Val Ser Leu Thr Gly Phe Leu
        35                  40                  45

Met Leu Glu Ile Val Leu Gly Leu Gly Ser Asn Leu Thr Val Leu Val
    50                  55                  60

Leu Tyr Cys Met Lys Ser Asn Leu Ile Asn Ser Val Ser Asn Ile Ile
65                  70                  75                  80

Thr Met Asn Leu His Val Leu Asp Val Ile Ile Cys Val Gly Cys Ile
                85                  90                  95

Pro Leu Thr Ile Val Ile Leu Leu Leu Ser Leu Glu Ser Asn Thr Ala
            100                 105                 110

Leu Ile Cys Cys Phe His Glu Ala Cys Val Ser Phe Ala Ser Val Ser
        115                 120                 125

Thr Ala Ile Asn Val Phe Ala Ile Thr Leu Asp Arg Tyr Asp Ile Ser
    130                 135                 140

Val Lys Pro Ala Asn Arg Ile Leu Thr Met Gly Arg Ala Val Met Leu
145                 150                 155                 160

Met Ile Ser Ile Trp Ile Phe Ser Phe Phe Ser Phe Leu Ile Pro Phe
                165                 170                 175

Ile Glu Val Asn Phe Phe Ser Leu Gln Ser Gly Asn Thr Trp Glu Asn
            180                 185                 190

Lys Thr Leu Leu Cys Val Ser Thr Asn Glu Tyr Tyr Thr Glu Leu Gly
        195                 200                 205

Met Tyr Tyr His Leu Leu Val Gln Ile Pro Ile Phe Phe Phe Thr Val
    210                 215                 220

Val Val Met Leu Ile Thr Tyr Thr Lys Ile Leu Gln Ala Leu Asn Ile
225                 230                 235                 240

Arg Ile Gly Thr Arg Phe Ser Thr Gly Gln Lys Lys Lys Ala Arg Lys
                245                 250                 255

Lys Lys Thr Ile Ser Leu Thr Thr Gln His Glu Ala Thr Asp Met Ser
            260                 265                 270

Gln Ser Ser Gly Gly Arg Asn Val Val Phe Gly Val Arg Thr Ser Val
        275                 280                 285

Ser Val Ile Ile Ala Leu Arg Arg Ala Val Lys Arg His Arg Glu Arg
    290                 295                 300

Arg Glu Arg Gln Lys Arg Val Phe Arg Met Ser Leu Leu Ile Ile Ser
305                 310                 315                 320

Thr Phe Leu Leu Cys Trp Thr Pro Ile Ser Val Leu Asn Thr Thr Ile
                325                 330                 335

Leu Cys Leu Gly Pro Ser Asp Leu Leu Val Lys Leu Arg Leu Cys Phe
            340                 345                 350

Leu Val Met Ala Tyr Gly Thr Thr Ile Phe His Pro Leu Leu Tyr Ala
        355                 360                 365

Phe Thr Arg Gln Lys Phe Gln Lys Val Leu Lys Ser Lys Met Lys Lys
    370                 375                 380

Arg Val Val Ser Ile Val Glu Ala Asp Pro Leu Pro Asn Asn Ala Val
385                 390                 395                 400

Ile His Asn Ser Trp Ile Asp Pro Lys Arg Asn Lys Lys Ile Thr Phe
                405                 410                 415

Glu Asp Ser Glu Ile Arg Glu Lys Cys Leu Val Pro Gln Val Val Thr
            420                 425                 430

Asp

<210>  3
<211>  3669
<212>  DNA
<213>  Homo sapiens

<220>
<221>  CDS
<222>  (18)..(917)
<223>  PEX19

<400>  3
aagtcggagg tagcaag atg gcc gcc gct gag gaa ggc tgt agt gtc ggg        50
                   Met Ala Ala Ala Glu Glu Gly Cys Ser Val Gly
                   1               5                   10

gcc gaa gcg gac agg gaa ttg gag gag ctt ctg gaa agt gct ctt gat       98
Ala Glu Ala Asp Arg Glu Leu Glu Glu Leu Leu Glu Ser Ala Leu Asp
            15                  20                  25

gat ttc gat aaa gcc aaa ccc tcc cca gca ccc cct tct acc acc acg      146
Asp Phe Asp Lys Ala Lys Pro Ser Pro Ala Pro Pro Ser Thr Thr Thr
        30                  35                  40

gcc cct gat gct tcg ggg ccc cag aag aga tcg cca gga gac act gcc      194
Ala Pro Asp Ala Ser Gly Pro Gln Lys Arg Ser Pro Gly Asp Thr Ala
    45                  50                  55

aaa gat gcc ctc ttc gct tcc caa gag aag ttt ttc cag gaa cta ttc      242
Lys Asp Ala Leu Phe Ala Ser Gln Glu Lys Phe Phe Gln Glu Leu Phe
60                  65                  70                  75

gac agt gaa ctg gct tcc caa gcc act gcg gag ttc gag aag gca atg      290
Asp Ser Glu Leu Ala Ser Gln Ala Thr Ala Glu Phe Glu Lys Ala Met
                80                  85                  90

aag gag ttg gct gag gaa gaa ccc cac ctg gtg gag cag ttc caa aag      338
Lys Glu Leu Ala Glu Glu Glu Pro His Leu Val Glu Gln Phe Gln Lys
            95                  100                 105

ctc tca gag gct gca ggg aga gtg ggc agt gat atg acc tcc caa caa      386
Leu Ser Glu Ala Ala Gly Arg Val Gly Ser Asp Met Thr Ser Gln Gln
        110                 115                 120

gaa ttc act tct tgc cta aag gaa aca cta agt gga tta gcc aaa aat      434
Glu Phe Thr Ser Cys Leu Lys Glu Thr Leu Ser Gly Leu Ala Lys Asn
    125                 130                 135

gcc act gac ctt cag aac tcc agc atg tcg gaa gaa gag ctg acc aag      482
Ala Thr Asp Leu Gln Asn Ser Ser Met Ser Glu Glu Glu Leu Thr Lys
140                 145                 150                 155

gcc atg gag ggg cta ggc atg gac gaa ggg gat ggg gaa ggg aac atc      530

Ala Met Glu Gly Leu Gly Met Asp Glu Gly Asp Gly Glu Gly Asn Ile
                160                 165                 170

ctc ccc atc atg cag agt att atg cag aac cta ctc tcc aag gat gtg      578
Leu Pro Ile Met Gln Ser Ile Met Gln Asn Leu Leu Ser Lys Asp Val
            175                 180                 185

ctg tac cca tca ctg aag gag atc aca gaa aag tat cca gaa tgg ttg      626
Leu Tyr Pro Ser Leu Lys Glu Ile Thr Glu Lys Tyr Pro Glu Trp Leu
        190                 195                 200

cag agt cat cgg gaa tct cta cct cca gag cag ttt gaa aaa tat cag      674
Gln Ser His Arg Glu Ser Leu Pro Pro Glu Gln Phe Glu Lys Tyr Gln
    205                 210                 215

gag cag cac agc gtc atg tgc aaa ata tgt gag cag ttt gag gca gag      722
Glu Gln His Ser Val Met Cys Lys Ile Cys Glu Gln Phe Glu Ala Glu
220                 225                 230                 235

acc ccc aca gac agt gaa acc act caa aag gct cgt ttt gag atg gtg      770
Thr Pro Thr Asp Ser Glu Thr Thr Gln Lys Ala Arg Phe Glu Met Val
                240                 245                 250

ctg gat ctt atg cag cag cta caa gat tta ggc cat cct cca aaa gag      818
Leu Asp Leu Met Gln Gln Leu Gln Asp Leu Gly His Pro Pro Lys Glu
            255                 260                 265

ctg gct gga gag atg cct cct ggc ctc aac ttt gac ctg gat gcc ctc      866
Leu Ala Gly Glu Met Pro Pro Gly Leu Asn Phe Asp Leu Asp Ala Leu
        270                 275                 280

aat ctt tcg ggc cca cca ggt gcc agt ggt gaa cag tgt ctg atc atg      914
Asn Leu Ser Gly Pro Pro Gly Ala Ser Gly Glu Gln Cys Leu Ile Met
    285                 290                 295

tga aacacaacac gttttcctct ctgagtccca gctatgggga acatctggag          967

tcagcagaac cattgggacc tgaggcagga gtgtcacctg cgggagaagt ctgcccgctg   1027

ccctctgtca tcccattcaa gattgtgcca taccagctga ggtttttcct ctgtctctct   1087

aggaataggg tctgtttcac aggccatttc tgtgaaccct actccattgt ggtttctgcc   1147

actatcaaag ttccagctac ctgcaaggtg aaggaaggca tcccttttgg ggcatgcact   1207

ttctttcctt tctcaaaata atgttatatg tggccacact gatgttcacc tttacgtcca   1267

gggtctttgt gccttgtctc tactccctct cttggatctg gggaggaggg gcagagacct   1327

gggactctgt atttctatag ttctcctggc agagcctttg agaatgggga gaaacagcct   1387

gggctggggc tacaggtctg tcactatgct ctcttgcctt cagacagacc attctgaatt   1447

ctctaaaggg aaaggctttt tgcatctaat cacaatagag ttgaaagaga ggccttagga   1507

ttctcctctc tctaggtgct gagccctcac ctccctgttc caggctgaga actcaaatgg   1567

ttaccctgct tcttcctaca atgctgtgtg atatgggtga acccagcccc tgaccttcct   1627

ctatcccctg cccatcctcc cttttacctc ctctcttttt taaacacctg tttatcccaa   1687

cctttttgag ctcaagctgt gataaagaag ggcccatcct atttcccctc atctagtcca 1747

tttacgattc tcactgactc cccgtcttcc tggcagacac aaataaaccc agtgtcaggt 1807

ctaggaaatt aatggctatt cttccccaga tacattctgg cttatttgag atacatgatt 1867

ctcttagaat cctgtccctt ggttcaggaa agtagcttgg aaaaggagta ggggtatagc 1927

ttgggtccct tttcctgcaa ggccccatgg ggcagaatat aataaatatt ctgagtgagg 1987

agtgtggtct ttttctgatc ttcctcagct tccgtaagtt gcagagtgag gtatattagg 2047

agactagttc tacacaatat tgtaatgctg ggttccatca acacccacct tccacaactc 2107

agtctgcacc tcagttggca aaggagactg gatggccatc tttcctcatg ttcccttgag 2167

tatttcaatg tagaaagccc ttcaagtggt attatatttt aaccttttac attattgtta 2227

ttaatgttag taatatattg ttatgttttc taaattattt ttctttaagc tgacgtggct 2287

ttttttctgt ggctcccagt gggtctacgg accttggctg acatatgttg gtaggtactc 2347

tggtcagctc agctggctgt cctggttcac tcagaagata agtctctcca aagcaaattc 2407

acatgcatta tgagtcgctt tgagcttctg acatgtcact tgccccgagg ttaaaacttt 2467

tcaccccttg aagaccttac atgttttatg gtattggtga ggaaggaaat gttctcaagg 2527

tctcaggcta tttgggaaat tccaactcct ataccttacc agagcatgga agagcccaga 2587

tctgaatgta aaacgtctct gttctgccag agatggaaaa aatacaggta tacttgtgat 2647

atagtcatgg ggcttcagtg tcactatttt ctccttaaag ctccagccaa aaactggaca 2707

aggatagaga ggaggaggga agaacaaaag agcccttctc tatgaacctt gtgccttctg 2767

tcctaccagt tttcttttac agattctcac ttctgctagc ctagccaggg cttactccag 2827

gaatctaaat agatgcccta gtccacttta tctttgttcc caaggcactc atttttattt 2887

tgattttgat tgaatgtgag caggttgacc tcaggtcaca ctttgttcca aaaacttttg 2947

gaattattcc aggacttgtg gtggagttat ggtactctag ggcagtcttt ctcaaactat 3007

gtatggtaaa ggaccaggtt ttttgttttc cagtccttca cttatcaata tgcattccta 3067

ttgccgatga caggtatgga gttcacactg tgtgctgccg acccggcaag tttgacagca 3127

cccaaactgg ccagactgtt ctgtaggtta agtccattga tcatgtactt ggatatcaca 3187

gcaacattga aatgctaaaa agtttttaaa cactctcaat ttctaattca ccatgtcaca 3247

gactggtgaa aaaaaaaaaa aggtgttcac tgaccagcac aagtctgcag atcatctttg 3307

agtagcactg ttttggggcc ctcggtctct ctgaagaccc tagcagaact gatacctacc 3367

tgtatctctt gttctctcct atttgagttt cacttccaga gaacttgttc ttcagcaaga 3427

atgtgtcact agtaaggaca tctctagcat ttctctagcc ttccttttct gctgctcaaa 3487

aataatcgtt acaaagctta ggtttaagct gtatatgaaa tatttatgcg actctcaaac 3547

tttaaaggag ttgctccttt gttccaaaat taaatgtgtt agataaattt gtgattgtat   3607

gggtggcttc atgaattaag aattgaatta atacagactt tttgataata aaaaaaaaaa   3667

aa                                                                  3669

<210>  4
<211>  299
<212>  PRT
<213>  Homo sapiens

<400>  4

Met Ala Ala Ala Glu Glu Gly Cys Ser Val Gly Ala Glu Ala Asp Arg
1               5                   10                  15

Glu Leu Glu Glu Leu Leu Glu Ser Ala Leu Asp Asp Phe Asp Lys Ala
            20                  25                  30

Lys Pro Ser Pro Ala Pro Pro Ser Thr Thr Thr Ala Pro Asp Ala Ser
        35                  40                  45

Gly Pro Gln Lys Arg Ser Pro Gly Asp Thr Ala Lys Asp Ala Leu Phe
    50                  55                  60

Ala Ser Gln Glu Lys Phe Phe Gln Glu Leu Phe Asp Ser Glu Leu Ala
65                  70                  75                  80

Ser Gln Ala Thr Ala Glu Phe Glu Lys Ala Met Lys Glu Leu Ala Glu
                85                  90                  95

Glu Glu Pro His Leu Val Glu Gln Phe Gln Lys Leu Ser Glu Ala Ala
            100                 105                 110

Gly Arg Val Gly Ser Asp Met Thr Ser Gln Gln Glu Phe Thr Ser Cys
        115                 120                 125

Leu Lys Glu Thr Leu Ser Gly Leu Ala Lys Asn Ala Thr Asp Leu Gln
    130                 135                 140

Asn Ser Ser Met Ser Glu Glu Glu Leu Thr Lys Ala Met Glu Gly Leu
145                 150                 155                 160

Gly Met Asp Glu Gly Asp Gly Glu Gly Asn Ile Leu Pro Ile Met Gln
                165                 170                 175

Ser Ile Met Gln Asn Leu Leu Ser Lys Asp Val Leu Tyr Pro Ser Leu
            180                 185                 190

Lys Glu Ile Thr Glu Lys Tyr Pro Glu Trp Leu Gln Ser His Arg Glu
        195                 200                 205

Ser Leu Pro Pro Glu Gln Phe Glu Lys Tyr Gln Glu Gln His Ser Val
    210                 215                 220

Met Cys Lys Ile Cys Glu Gln Phe Glu Ala Glu Thr Pro Thr Asp Ser
225                 230                 235                 240

Glu Thr Thr Gln Lys Ala Arg Phe Glu Met Val Leu Asp Leu Met Gln
                245                 250                 255

Gln Leu Gln Asp Leu Gly His Pro Pro Lys Glu Leu Ala Gly Glu Met
            260                 265                 270

Pro Pro Gly Leu Asn Phe Asp Leu Asp Ala Leu Asn Leu Ser Gly Pro
        275                 280                 285

Pro Gly Ala Ser Gly Glu Gln Cys Leu Ile Met
    290                 295

<210>  5
<211>  5231
<212>  DNA
<213>  Homo sapiens

<220>
<221>  CDS
<222>  (331)..(2208)
<223>  GRHL2

<400>  5
agaaagttac ctgtggccgc ccaagtccgc cactttctgc tctgtgtctg cccattgcca      60
cgatccagga ggactccgcg ccgcccggcc gcctccgagc tcgggcccca tgtgaggggc     120
ccccccttat cccacctttc cggctaggtg agggcgcgag cgggcgagcg agcgagagtg     180
gtgagggggg acggaaaagc agaattacct gtagctcttg ttctgccatc tcgggcgctc     240
tcacacacct tcacctgcac agacttgaaa gtccagtttc accagaggct gaggctccag     300
gaaaagcgga gcaagttcat tggatcaaac atg tca caa gag tcg gac aat aat     354
aaa aga cta gtg gcc tta gtg ccc atg ccc agt gac cct cca ttc aat     402
acc cga aga gcc tac acc agt gag gat gaa gcc tgg aag tca tac ttg     450
gag aat ccc ctg aca gca gcc acc aag gcc atg atg agc att aat ggt      498
gat gag gac agt gct gct gcc ctc ggc ctg ctc tat gac tac tac aag      546
gtt cct cga gac aag agg ctg ctg tct gta agc aaa gca agt gac agc      594
caa gaa gac cag gag aaa aga aac tgc ctt ggc acc agt gaa gcc cag      642
agt aat ttg agt gga gga gaa aac cga gtg caa gtc cta aag act gtt      690
cca gtg aac ctt tcc cta aat caa gat cac ctg gag aat tcc aag cgg      738
gaa cag tac agc atc agc ttc ccc gag agc tct gcc atc atc ccg gtg      786
tcg gga atc acg gtg gtg aaa gct gaa gat ttc aca cca gtt ttc atg      834
gcc cca cct gtg cac tat ccc cgg gga gat ggg gaa gag caa cga gtg      882
gtt atc ttt gaa cag act cag tat gac gtg ccc tcg ctg gcc acc cac      930
agc gcc tat ctc aaa gac gac cag cgc agc act ccg gac agc aca tac      978
agc gag agc ttc aag gac gca gcc aca gag aaa ttt cgg agt gct tca     1026
gtt ggg gct gag gag tac atg tat gat cag aca tca agt ggc aca ttt     1074
cag tac acc ctg gaa gcc acc aaa tct ctc cgt cag aag cag ggg gag     1122
ggc ccc atg acc tac ctc aac aaa gga cag ttc tat gcc ata aca ctc     1170
agc gag acc gga gac aac aaa tgc ttc cga cac ccc atc agc aaa gtc      1218
agg agt gtg gtg atg gtg gtc ttc agt gaa gac aaa aac aga gat gaa      1266
cag ctc aaa tac tgg aaa tac tgg cac tct cgg cag cat acg gcg aag      1314
cag agg gtc ctt gac att gcc gat tac aag gag agc ttt aat acg att      1362
gga aac att gaa gag att gca tat aat gct gtt tcc ttt acc tgg gac      1410
gtg aat gaa gag gcg aag att ttc atc acc gtg aat tgc ttg agc aca      1458
gat ttc tcc tcc caa aaa ggg gtg aaa gga ctt cct ttg atg att cag      1506
att gac aca tac agt tat aac aat cgt agc aat aaa ccc att cat aga      1554
gct tat tgc cag atc aag gtc ttc tgt gac aaa gga gca gaa aga aaa      1602
atc cga gat gaa gag cgg aag cag aac agg aag aaa ggg aaa ggc cag      1650
gcc tcc caa act caa tgc aac agc tcc tct gat ggg aag ttg gct gcc      1698
ata cct tta cag aag aag agt gac atc acc tac ttc aaa acc atg cct      1746
gat ctc cac tca cag cca gtt ctc ttc ata cct gat gtt cac ttt gca      1794
aac ctg cag agg acc gga cag gtg tat tac aac acg gat gat gaa cga      1842
gaa ggt ggc agt gtc ctt gtt aaa cgg atg ttc cgg ccc atg gaa gag      1890
gag ttt ggt cca gtg cct tca aag cag atg aaa gaa gaa ggg aca aag     1938
cga gtg ctc ttg tac gtg agg aag gag act gac gat gtg ttc gat gca     1986
ttg atg ttg aag tct ccc aca gtg aag ggc ctg atg gaa gcg ata tct     2034
gag aaa tat ggg ctg ccc gtg gag aag ata gca aag ctt tac aag aaa     2082
agc aaa aaa ggc atc ttg gtg aac atg gat gac aac atc atc gag cac     2130
tac tcg aac gag gac acc ttc atc ctc aac atg gag agc atg gtg gag     2178
ggc ttc aag gtc acg ctc atg gaa atc tag ccctgggttt ggcatccgct       2228
ttggctggag ctctcagtgc gttcctccct gagagagaca gaagccccag ccccagaacc   2288
tggagaccca tctcccccat ctcacaactg ctgttacaag accgtgctgg ggagtggggc   2348
aagggacagg ccccactgtc ggtgtgcttg gcccatccac tggcacctac cacggagctg   2408
aagcctgagc ccctcaggaa ggtgccttag gcctgttgga ttcctattta ttgcccacct    2468
tttcctggag cccaggtcca ggcccgccag gactctgcag gtcactgcta gctccagatg   2528
agaccgtcca gcgttccccc ttcaagagaa acactcatcc cgaacagcct aaaaaattcc   2588
catcccttct ctctcacccc tccatatcta tctcccgagt ggctggacaa aatgagctac   2648
gtctgggtgc agtagttata ggtggggcaa gaggtggatg cccactttct ggtcagacac   2708
ctttaggttg ctctggggaa ggctgtcttg ctaaatacct ccagggttcc cagcaagtgg    2768
ccaccaggcc ttgtacagga agacattcag tcaccgtgta attagtaaca cagaaagtct   2828
gcctgtctgc attgtacata gtgtttataa tattgtaata atatatttta cctgtggtat   2888
gtgggcatgt ttactgccac tggcctagag gagacacaga cctggagacc gttttaatgg   2948
gggtttttgc ctctgtgcct gttcaagaga cttgcagggc taggtagagg gcctttggga   3008
tgttaaggtg actgcagctg atgccaagat ggactctgca atgggcatac ctgggggctc   3068
gttccctgtc cccagaggaa gccccctctc cttctccatg ggcatgactc tccttcgagg    3128
ccaccacgtt tatctcacaa tgatgtgttt tgcttgactt tccctttgcg ctgtctcgtg   3188
ggaaaggtca ttctgtctga gaccccagct ccttctccag ctttggctgc gggcatggcc 3248
tgagctttct ggagagcctc tgcagggggt ttgccatcag ggccctgtgg ctgggtctgc 3308
tgcagagctc cttggctatc aggagaatcc tggacactgt actgtgcctc ccagtttaca 3368
aacacgccct tcatctcaag tggcccttta aaaggcctgc tgccatgtga gagctgtgaa 3428
cagctcagct ctgagtcggc aggctggggc ttcctcctgg gccaccagat ggaaaggggg 3488
tattgtttgc ctcactcctg gatgctgcgt tttaaggaag tgagtgagaa agaatgtgcc 3548
aagatacctg gctcctgtga aaccagcctc aggagggaaa ctgggagaga gaagctgtgg 3608
tctcctgcta catgccctgg gagctggaag agaaaaacac tcccctaaac aatcgcaaaa 3668
tgatgaacca tcatgggcca ctgttctctt tgaggggaca ggtttagggg tttgcgttcg 3728
cccttgtggg ctgaagcact agctttttgg tagctagaca catcctgcac ccaaaggttc 3788
tctacaaagg cccagatttg tttgtaaagc actttgactc ttacctggag gcccgctctc 3848
taagggcttc ctgcgctccc acctcatctg tccctgagat gcagagcagg atggagggtc 3908
tgcttctagc tcagctgttt ctccttgagg ttgcggagga attgaattga atgggacaga 3968
gggcaggtgc tgtggccaag aagatctccg agcagcagtg acggggcacc ttgctgtgtg 4028
tcctctgggc atgttaaccc ttctgtgggg ccaaaggttt gcatcgtgga tccagctgtg 4088
ctccagtctg tcccctcctc ctccactctg actgccacgc cccggaccag cagcttgggg 4148
accctccagg gtactaatgg ggctctgttc tgagatggac aaattcagtg ttggaaatac 4208
atgttgtact atgcacttcc catgctccta gggttaggaa tagtttcaaa catgattggc 4268
agacataaca acggcaaata ctcggactgg ggcataggac tccagagtag gaaaaagaca 4328
aaagatttgg cagcctgaca caggcaacct acccctctct ctccagcctc tttatgaaac 4388
tgtttgtttg ccagtcctgc cctaaggcag aagatgaatt gaagatgctg tgcatgtttc 4448
ctaagtcctt gagcaatcat ggtggtgaca attgccacaa gggatatgag gccagtgcca 4508
ccagagggtg gtgccaagtg ccacatccct tccgatccat tcccctctgc atcctcggag 4568
caccccagtt tgcctttgat gtgtccgctg tgtatgttag ctgaactttg atgagcaaaa 4628
tttcctgagc gaaacactcc aaagagatag gaaaacttgc cgcctcttct tttttgtccc 4688
ttaatcaaac tcaaataagc ttaaaaaaaa tccatggaag atcatggaca tgtgaaatga 4748
gcattttttt cttttttttt tttaacaaag tctgaactga acagaacaag actttttcct 4808
catacatctc caaattgttt aaacttactt tatgagtgtt tgtttagaag ttcggaccaa 4868
cagaaaaatg cagtcagatg tcatcttgga attggtttct aaaagagtaa ggcatgtccc 4928
tgcccagaaa cttaggaagc atgaaataaa tcaaatgttt attttccttc ttatttaaaa 4988
tcatgcaaat gcaacagaaa tagagggttt gtgccaaatg ctatgaacgg ccctttctta 5048
aagacaagca agggagattg atatatgtac aatttgctct catgttttaa aaaaaaaaag 5108
gtaaatgtaa cttaatagtt ttgtaaatgg gagaggggga atctataaac tataaataca 5168
gttattttat tttttgtaca tttttaagga gaaaaaataa atattcataa cataagagta 5228
aaa 5231

<210> 6
<211> 625
<212> PRT
<213> Homo sapiens

<400> 6

<210>  7
<211>  3033
<212>  DNA
<213>  Homo sapiens

<220>
<221>  CDS
<222>  (180)..(935)

<400>  7
gaaggctgcc tcgctggtcc gaattcggtg gcgccacgtc cgcccgtctc cgccttctgc      60

atcgcggctt cggcggcttc cacctagaca cctaacagtc gcggagccgg ccgcgtcgtg     120

agggggtcgg cacggggagt cgggcggtct tgtgcatctt ggctacctgt gggtcgaag      179

atg tcg gac atc gga gac tgg ttc agg agc atc ccg gcg atc acg cgc       227
Met Ser Asp Ile Gly Asp Trp Phe Arg Ser Ile Pro Ala Ile Thr Arg
1               5                   10                  15

tat tgg ttc gcc gcc acc gtc gcc gtg ccc ttg gtc ggc aaa ctc ggc       275
Tyr Trp Phe Ala Ala Thr Val Ala Val Pro Leu Val Gly Lys Leu Gly
            20                  25                  30

ctc atc agc ccg gcc tac ctc ttc ctc tgg ccc gaa gcc ttc ctt tat       323
Leu Ile Ser Pro Ala Tyr Leu Phe Leu Trp Pro Glu Ala Phe Leu Tyr
        35                  40                  45

cgc ttt cag att tgg agg cca atc act gcc acc ttt tat ttc cct gtg       371
Arg Phe Gln Ile Trp Arg Pro Ile Thr Ala Thr Phe Tyr Phe Pro Val
    50                  55                  60

ggt cca gga act gga ttt ctt tat ttg gtc aat tta tat ttc tta tat       419
Gly Pro Gly Thr Gly Phe Leu Tyr Leu Val Asn Leu Tyr Phe Leu Tyr
65                  70                  75                  80

cag tat tct acg cga ctt gaa aca gga gct ttt gat ggg agg cca gca       467
Gln Tyr Ser Thr Arg Leu Glu Thr Gly Ala Phe Asp Gly Arg Pro Ala
                85                  90                  95

gac tat tta ttc atg ctc ctc ttt aac tgg att tgc atc gtg att act       515
Asp Tyr Leu Phe Met Leu Leu Phe Asn Trp Ile Cys Ile Val Ile Thr
            100                 105                 110

ggc tta gca atg gat atg cag ttg ctg atg att cct ctg atc atg tca       563
Gly Leu Ala Met Asp Met Gln Leu Leu Met Ile Pro Leu Ile Met Ser
        115                 120                 125

gta ctt tat gtc tgg gcc cag ctg aac aga gac atg att gta tca ttt       611
Val Leu Tyr Val Trp Ala Gln Leu Asn Arg Asp Met Ile Val Ser Phe
    130                 135                 140

tgg ttt gga aca cga ttt aag gcc tgc tat tta ccc tgg gtt atc ctt       659
Trp Phe Gly Thr Arg Phe Lys Ala Cys Tyr Leu Pro Trp Val Ile Leu
145                 150                 155                 160

gga ttc aac tat atc atc gga ggc tcg gta atc aat gag ctt att gga       707
Gly Phe Asn Tyr Ile Ile Gly Gly Ser Val Ile Asn Glu Leu Ile Gly
                165                 170                 175

aat ctg gtt gga cat ctt tat ttt ttc cta atg ttc aga tac cca atg       755
Asn Leu Val Gly His Leu Tyr Phe Phe Leu Met Phe Arg Tyr Pro Met
            180                 185                 190

gac ttg gga gga aga aat ttt cta tcc aca cct cag ttt ttg tac cgc       803
Asp Leu Gly Gly Arg Asn Phe Leu Ser Thr Pro Gln Phe Leu Tyr Arg

195                 200                 205

tgg ctg ccc agt agg aga gga gga gta tca gga ttt ggt gtg ccc cct      851
Trp Leu Pro Ser Arg Arg Gly Gly Val Ser Gly Phe Gly Val Pro Pro
    210                 215                 220

gct agc atg agg cga gct gct gat cag aat ggc gga ggc ggg aga cac      899
Ala Ser Met Arg Arg Ala Ala Asp Gln Asn Gly Gly Gly Gly Arg His
225                 230                 235                 240

aac tgg ggc cag ggc ttt cga ctt gga gac cag tga aggggcggcc           945
Asn Trp Gly Gln Gly Phe Arg Leu Gly Asp Gln
                245                 250

tcgggcagcc gctcctctca agccacattt cctcccagtg ctgggtgcac ttaacaactg   1005

cgttctggct aacactgttg gacctgaccc acactgaatg tagtctttca gtacgagaca   1065

aagtttctta aatcccgaag aaaaatataa gtgttccaca agtttcacga ttctcattca   1125

agtccttact gctgtgaaga acaaatacca actgtgcaaa ttgcaaaact gactacattt   1185

tttggtgtct tctcttctcc cctttccgtc tgaataatgg gttttagcgg gtcctaatct   1245

gctggcattg agctggggct gggtcaccaa acccttccca aaaggacctt atctctttct   1305

tgcacacatg cctctctccc acttttccca acccccacat ttgcaactag aaaaagttgc   1365

ccataaaatt gctctgccct tgacaggttc tgttatttat tgacttttgc caaggctggt   1425

cacaacaatc atattcacgt tattttcccc ttttggtggc agaactgtta ccaatagggg   1485

gagaagacag ccacggatga agcgtttctc agcttttgga attgcttcga ctgacatccg   1545

ttgttaaccg tttgccactc ttcagatatt ttttataaaa aaagtaccac tgagttcatg   1605

agggccacag attggttatt aatgagatac gagggttggt gctgggtgtt tgtttcctga   1665

gctaagtgat caagactgta gtggagttgc agctaacatg ggttaggttt aaaccatggg   1725

ggatgcaccc ctttgcgttt catatgtagc cctactggct ttgtgtagct ggagtagttg   1785

ggttgctttg tgttaggagg atccagatca tgttggctac agggagatgc tctctttgag   1845

aggtcctggg cattgattcc catttcaatc tcattctgga tatgtgttca ttgagtaaag   1905

gaggagagac cctcatacgc tatttaaatg tcactttttt gcctatcccc cgttttttgg   1965

tcatgtttca attaattgtg aggaaggcgc agctcctctc tgcacgtaga tcatttttta   2025

aagctaatgt aagcacatct aagggaataa catgatttaa ggttgaaatg gctttagaat   2085

catttgggtt tgagggtgtg ttattttgag tcatgaatgt acaagctctg tgaatcagac   2145

cagcttaaat acccacacct ttttttcgta ggtgggcttt tcctatcaga gcttggctca   2205

taaccaaata aagttttttg aaggccatgg cttttcacac agttatttta ttttatgacg   2265

ttatctgaaa gcagactgtt aggagcagta ttgagtggct gtcacacttt gaggcaacta   2325

aaaaggcttc aaacgttttg atcagtttct tttcaggaaa cattgtgctc taacagtatg   2385

actattcttt cccccactct taaacagtgt gatgtgtgtt atcctaggaa atgagagttg    2445

gcaaacaact tctcattttg aatagagttt gtgtgtactt ctccatattt aatttatatg    2505

ataaaatagg tggggagagt ctgaacctta actgtcatgt tttgttgttc atctgtggcc    2565

acaataaagt ttacttgtaa aattttagag gccattactc caattatgtt gcacgtacac    2625

tcattgtaca ggcgtggaga ctcattgtat gtataagaat atttctgaca gtgagtgacc    2685

cggagtctct ggtgtaccct cttaccagtc agctgcctgc gagcagtcat tttttcctaa    2745

aggtttacaa gtatttagaa cttttcagtt cagggcaaaa tgttcatgaa gttattcctc    2805

ttaaacatgg ttaggaagct gatgacgtta ttgattttgt ctggattatg tttctggaat    2865

aattttacca aaacaagcta tttgagtttt gacttgacaa ggcaaaacat gacagtggat    2925

tctctttaca aatggaaaaa aaaaatcctt attttgtata aaggacttcc ctttttgtaa    2985

actaatcctt tttattggta aaaattgtaa attaaaatgt gcaacttg                3033

<210>  8
<211>  251
<212>  PRT
<213>  Homo sapiens

<400>  8

Met Ser Asp Ile Gly Asp Trp Phe Arg Ser Ile Pro Ala Ile Thr Arg
1               5                   10                  15

Tyr Trp Phe Ala Ala Thr Val Ala Val Pro Leu Val Gly Lys Leu Gly
            20                  25                  30

Leu Ile Ser Pro Ala Tyr Leu Phe Leu Trp Pro Glu Ala Phe Leu Tyr
        35                  40                  45

Arg Phe Gln Ile Trp Arg Pro Ile Thr Ala Thr Phe Tyr Phe Pro Val
    50                  55                  60

Gly Pro Gly Thr Gly Phe Leu Tyr Leu Val Asn Leu Tyr Phe Leu Tyr
65                  70                  75                  80

Gln Tyr Ser Thr Arg Leu Glu Thr Gly Ala Phe Asp Gly Arg Pro Ala
                85                  90                  95

Asp Tyr Leu Phe Met Leu Leu Phe Asn Trp Ile Cys Ile Val Ile Thr
            100                 105                 110

Gly Leu Ala Met Asp Met Gln Leu Leu Met Ile Pro Leu Ile Met Ser
        115                 120                 125

Val Leu Tyr Val Trp Ala Gln Leu Asn Arg Asp Met Ile Val Ser Phe
    130                 135                 140

Trp Phe Gly Thr Arg Phe Lys Ala Cys Tyr Leu Pro Trp Val Ile Leu
145                 150                 155                 160

Gly Phe Asn Tyr Ile Ile Gly Gly Ser Val Ile Asn Glu Leu Ile Gly
                165                 170                 175

Asn Leu Val Gly His Leu Tyr Phe Phe Leu Met Phe Arg Tyr Pro Met
            180                 185                 190

Asp Leu Gly Gly Arg Asn Phe Leu Ser Thr Pro Gln Phe Leu Tyr Arg
        195                 200                 205

Trp Leu Pro Ser Arg Arg Gly Gly Val Ser Gly Phe Gly Val Pro Pro
    210                 215                 220

Ala Ser Met Arg Arg Ala Ala Asp Gln Asn Gly Gly Gly Gly Arg His
225                 230                 235                 240

Asn Trp Gly Gln Gly Phe Arg Leu Gly Asp Gln
                245                 250

<210>  9
<211>  21
<212>  DNA
<213>  Artificial sequence

<220>
<223>  Survivin _ Forward

<400>  9
ccagatgacg accccataga g                                                21

<210>  10
<211>  23
<212>  DNA
<213>  Artificial sequence

<220>
<223>  Survivin _ Reverse

<400>  10
ttgttggttt cctttgcaat ttt                                              23

<210>  11
<211>  22
<212>  DNA

<213>  Artificial sequence

<220>
<223>  Survivin _ Probe

<400>  11
cattcgtccg gttgcgcttt cc                                                22


<210>  12
<211>  20
<212>  DNA
<213>  Artificial sequence

<220>
<223>  Survivin-2B_ Forward

<400>  12
aagaactggc ccttcttgga                                                   20


<210>  13
<211>  24
<212>  DNA
<213>  Artificial sequence

<220>
<223>  Survivin-2B _ Reverse

<400>  13
ccaagtgctg gtattacagg cgta                                              24


<210>  14
<211>  23
<212>  DNA
<213>  Artificial sequence

<220>
<223>  Survivin-2B _ Probe

<400>  14
actgccccac tgagaacgag cca                                               23


<210>  15
<211>  22
<212>  DNA
<213>  Artificial sequence

<220>
<223>  Survivin- delta Ex3 _ Forward

<400>  15
cccagtgttt cttctgcttc aa                                                22


<210>  16
<211>  24
<212>  DNA
<213>  Artificial sequence

<220>
<223>  Survivin- delta Ex3 _ Reverse

<400>  16
ttcttcgcag tttcctcaaa ttct                                              24


<210>  17
<211>  26
<212>  DNA
<213>  Artificial sequence

<220>
<223>  Survivin- delta Ex3 _ Probe

<400>  17
acgaccccat gcaaaggaaa ccaaca                                            26


<210>  18
<211>  21
<212>  DNA
<213>  Artificial sequence

<220>
<223>  Survivin-3B _ Forward

<400>  18
ccagatgacg accccataga g                                                 21


<210>  19
<211>  20
<212>  DNA
<213>  Artificial sequence

<220>
<223>  Survivin-3B _ Reverse

<400>  19
aagaactggc ccttcttgga                                                   20


<210>  20
<211>  22
<212>  DNA
<213>  Artificial sequence

<220>
<223>  Survivin-3B _ Probe

<400>  20
cattcgtccg gttgcgcttt cc                                                22


<210>  21
<211>  25
<212>  DNA
<213>  Artificial sequence

<220>
<223>  Survivin-2 alpha _ Forward

<400>  21
gctttgtttt gaactgagtt gtcaa                                          25


<210>  22
<211>  19
<212>  DNA
<213>  Artificial sequence

<220>
<223>  Survivin-2 alpha _ Reverse

<400>  22
gcaatgaggg tggaaagca                                                 19


<210>  23
<211>  35
<212>  DNA
<213>  Artificial sequence

<220>
<223>  Survivin-2 alpha _ Probe

<400>  23
agatttgagt tgcaaagaca cttagtatgg gaggg                               35


<210>  24
<211>  21
<212>  DNA
<213>  Artificial sequence

<220>
<223>  Caspase-3 _ Forward

<400>  24
ctggactgtg gcattgagac a                                              21


<210>  25
<211>  20
<212>  DNA
<213>  Artificial sequence

<220>
<223>  Caspase-3 _ Reverse

<400>  25
agtcggcctc catggtattt                                                20


<210>  26
<211>  26
<212>  DNA
<213>  Artificial sequence

<220>

<223>  Caspase-3 _ Probe

<400>  26
tggtgttgat gatgacatgg cgtgtc                                            26


<210>  27
<211>  26
<212>  DNA
<213>  Artificial sequence

<220>
<223>  Caspase-3s _ Forward

<400>  27
agaagtctaa ctggaaaacc caaact                                            26


<210>  28
<211>  20
<212>  DNA
<213>  Artificial sequence

<220>
<223>  Caspase-3s _ Reverse

<400>  28
caaagcgact ggatgaacca                                                   20


<210>  29
<211>  25
<212>  DNA
<213>  Artificial sequence

<220>
<223>  Caspase-3s _ Probe

<400>  29
attattcagg ttattattct tggcg                                             25